(19) **Europäisches Patentamt**
**European Patent Office**
**Office européen des brevets**

(11) **EP 1 708 108 B1**

(12) **EUROPEAN PATENT SPECIFICATION**

(45) Date of publication and mention
of the grant of the patent:
**02.09.2009 Bulletin 2009/36**

(51) Int Cl.:
***G06F 17/50*** *(2006.01)*

(21) Application number: **06005156.2**

(22) Date of filing: **14.03.2006**

(54) **Improvements in or relating to quantitative structure-activity relationships (QSAR) for the case of missing fragments**

Verbesserungen in der Formulierung von quantitativen Struktur-Aktivitäts-Beziehungen (QSAR) im Falle fehlender Fragmente

Améliorations de construction des relations quantitatives structure-activité (RQSA) en cas de fragments manquants

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.03.2005 GB 0506366**

(43) Date of publication of application:
**04.10.2006 Bulletin 2006/40**

(73) Proprietor: **ID Business Solutions Ltd.**
**Guildford**
**Surrey GU2 7QB (GB)**

(72) Inventors:
• **Kolossov, Evgueni**
**Maidenhead**
**Berkshire SL6 2RZ (GB)**
• **Stanforth, Robert**
**Cranleigh**
**Surrey GU6 7DJ (GB)**

(74) Representative: **Anderson, Angela Mary**
**MURGITROYD & COMPANY**
**Immeuble Atlantis**
**55 Allée Pierre Ziller**
**Sophia Antipolis**
**06560 Valbonne (FR)**

(56) References cited:
**US-A- 6 036 349**

• **E. KOLOSSOV, R. STANFORTH: "Assessment of Domain of Applicability for the Fragment-based QSAR Models" POSTER AT: UK QSAR AND CHEMOINFORMATICS SPRING MEETING, 13 April 2005 (2005-04-13), XP001247035**
• **EVGUENI KOLOSSOV: "The Quality of QSAR Models: Myth and Reality" PRESENTATION AT: UK QSAR AND CHEMOINFORMATICS SPRING MEETING, 13 April 2005 (2005-04-13), XP002380041**
• **GUHA RAJARSHI ET AL: "Determining the validity of a QSAR model - A classification approach" J. CHEM. INF. MODEL.; JOURNAL OF CHEMICAL INFORMATION AND MODELING JANUARY/FEBRUARY 2005, vol. 45, no. 1, November 2004 (2004-11), pages 65-73, XP002380042**
• **SHERIDAN ROBERT P ET AL: "Similarity to molecules in the training set is a good discriminator for prediction accuracy in QSAR" J. CHEM. INF. COMPUT. SCI.; JOURNAL OF CHEMICAL INFORMATION AND COMPUTER SCIENCES NOVEMBER/DECEMBER 2004, vol. 44, no. 6, November 2004 (2004-11), pages 1912-1928, XP002380043**
• **W. LINDBERG, J-A. PERSSON: "Partial Least-Squares Method for Spectrofluorimetric Analysis of Mixtures of Humic Acis and Ligninsulfonate" ANALYTICAL CHEMISTRY, vol. 55, no. 4, 1983, pages 643-648, XP002380044**
• **MANDEL JOHN: "REGRESSION ANALYSIS OF COLLINEAR DATA" J RES NATL BUR STAND (US) NOV-DEC 1985, vol. 90, no. 6, 20 May 1985 (1985-05-20), XP002380045**

- **ALEXANDER TROPSHA, PAOLA GRAMATICA , VIJAY K. GOMBAR: "The Importance of Being Earnest: Validation is the Absolute Essential for Successful Application and Interpretation of QSPR Models" QSAR & COMBINATORIAL SCIENCE, vol. 22, no. 1, April 2003 (2003-04), pages 69-77, XP002380046**
- **NFW EL TAYAR, HFW KARAJIANNIS, HFW WATERBEEMD: "Structure-lipophilicity relationships of peptides and peptidomimetics" AMINO ACIDS, vol. 8, 1995, pages 125-139, XP009066055**
- **D. B. KITCHEN; F. L. STAHURA; J. BAJORATH: "Computational Techniques for Diversity Analysis and Compound Classification" MINI REVIEWS IN MEDICINAL CHEMISTRY, vol. 4, no. 10, December 2004 (2004-12), pages 1029-1039, XP009066112**
- **C. AUER, M. COMBER, JOANNA S. JAWORSKA, C.J. VAN LEEUWEN: "Summary of a Workshop on Regulatory Acceptance of (Q)SARs for Human Health and Environmental Endpoints" ENVIRONMENTAL HEALTH PERSPECTIVES, vol. 111, no. 10, August 2003 (2003-08), pages 1358-1360, XP002380049**
- **N NIKOLOVA, J JAWORSKA: "Approaches to measure chemical similarity: a review" QSAR & COMBINATORIAL SCIENCE, vol. 22, no. 9,10, 2003, pages 1006-1026, XP002380050**

**Description**

This invention relates to Quantitative Structure activity relationships (QSAR) in particular, but not exclusively to the development and validation status of QSAR models.

QSAR represents a technique to model the relationships between the chemical structures and their biological or physico-chemical properties. The models and correlation techniques are used to predict *in silico* what properties a chemical structure might have. This enables new chemicals and drugs to be created which are more likely to meet certain criteria. For example, the prediction of a bad side effect can prevent a potential drug from the next stage of research and in this way, save money and resources. There are clearly many advantages in using this type of analysis to speed up the creation and/or manufacture of new chemicals and prevent potential drug failures at earlier stages of the creation or manufacture.

Some QSAR models are created using fragments or bits of a specific chemical compound. For example the fragments may be groups attached to say an organic compound such as: a nitro group, an ethyl group, etc. The fragments once identified are compared with fragments in the QSAR fragment model to determine a degree of correlation between the fragments and the activity.

A QSAR fragment-based model is the set of fragments and their contributions (weights). To predict the property/ activity for the structure in question, this structure needs to be split into the fragments. Then for each of the fragments the match needs to be found in the set of fragments in the model. The final prediction value for this structure will be the sum of contributions for all fragments. If there is no match in the model for a particular fragment, then the prediction actually fails. This problem in QSAR modelling is called 'problem of missing fragments'.

At present there are no clearly defined universal quantitative determinations to enable consistent regulatory principles for QSAR models. This is an issue which is currently under consideration by many, but particularly the Organisation for Economic Co-operation and Development (OECD). The main principles known as the Setubal principles are that a QSAR should:

a. have a defined endpoint;
b. take the form of an unambiguous algorithm;
c. have a defined domain of applicability;
d. have appropriate measure of goodness-of-fit, robustness and predictivity; and
e. have a mechanistic interpretation, if possible.

The principle which is causing the most problems is the principle of having a defined domain of applicability. The need to define an applicability domain takes account of the fact that QSARs are reductionist models and generally have a number of limitations. These limitations are in terms of the types of chemical structures, physicochemical properties and mechanisms of action for which the model can generate reliable predictions. It is thus an object to define the type of information necessary to define QSAR applicability domains and how to determine this.

A QSAR descriptor-based model is the set of descriptor contributions (weights). To predict a property/activity for the structure in question descriptor values are calculated and multiplied by the contribution values. Because the descriptor values are numerical, it is possible to have a definition for the domain: For example, if there is only one descriptor, then any value calculated for the structure in question which is lower than the lowest values or higher than the highest values (all structures in a training set equal the model) will indicate that this compound is outside the domain. The distance from the domain will be the distance between the values calculated for the structure in question and the lowest or highest value in the model. This approach cannot be used for fragment-based models because fragments unlike the descriptors are not numerical.

For descriptor-based models a set of well known methods can be applied for domain of applicability assessment. An example of this is disclosed in a paper by R.S. Sheridan et al. (See: R.S Sheridan, BP Fenston, VN Maiorov and SK Kearsley; Similarity of molecules in the training set is a good discriminator for prediction accuracy in QSAR. J. Chem. Inf. Comput.SCI., 2004(44), 1912-1928). In this method the most useful measures that were tested are:

(1) The similarity of the molecule to be predicted to the nearest molecule in the training set; and/or
(2) The numbers of neighbours in the training set, where neighbours are those more similar than a user chosen cut-off.

However fragment-based models tend to be more significant and produce more information for research than descriptor-based models, as previously identified. The methods identified above do not however work for fragment based models for the following reasons:

a. no account is taken of the number of fragments generated from the structure;
b. no account is taken of any similarities between fragments generated from the structure and fragments in the model; and
c. no measure of any distance from the domain is taken to indicate degree of error in any predictions.

One object of the present invention is to provide a method of determining domain of applicability for fragment-based QSAR model.
Another object of the present invention is to overcome at least some of the disadvantages of know QSAR modelling.
According to one aspect of the present invention there is provided a computer method for estimation of the distance from an application domain of a fragemnt based model by means of a fragment - based Quantitative Structure Activity Relationship (QSAR) model, the steps comprising:

identifying the fragments [$r_1$ to $r_n$] in a structure;
comparing the or each fragment with one or more fragments in the model;
if the or each fragment substantially matches a fragment in a model determining a first error measure between a contribution of the or each fragment and a contribution of the matching fragment in the model;
if the or each fragment does not substantially match a fragment in the model determining which fragment in the model is the most similar to the or each fragment and determining a second error measure based on the similarity between the fragment and the most similar fragment; and
combining the first error measure and the second error measure to generate a degree of separation between the structure and of the combined fragments in the model to estimate the distance from the structure to an application domain of said fragment based QSAR model.

Preferably the step of identifying the fragments ($r$) further comprises for the or each structure ($k$) determining matrix X of mapping count $m_r$ (for fragments r) for the structure k such that

$$X = [m_{kr}].$$

Advantageously the step of determining the first error measure comprises calculating an error weight coefficient $e_r$ in accordance with

$$e_r = E_{rr} \text{--------} 4$$

where

$$E = \left( X^T X - \frac{1}{N}\left(\sum_{k=1}^{N} \mathbf{x}_k\right)\left(\sum_{k=1}^{N} \mathbf{x}_k^T\right)\right)^{-1} \text{--------} 5$$

$N$ is the number of structures which have been trained on in the model;
$x_k=[m_{kr}]$ is the row of X containing the mappings for structure $k$.

Preferably the step of determining the second error measure comprises measuring the degree of dissimilarity ($1-T_r$) of the or each fragment from the most similar fragment in the model.
In one embodiment the step of combining comprises combining the error weight coefficient and the dissimilarity.
Preferable the distance of the structure from the domain of the QSAR fragment model ($d_{fm}$) is estimated in accordance with:

$$d_{fm}^2 = \frac{1}{N}\sum_{r=1}^{N}(1-T_r)m_r^2 e_r$$

where $T_r$ is the measure of similarly and $m_r$ is the mapping count for fragment r.

According to a second aspect of the present invention there is provided apparatus for estimating the distance from an application domain Quantitative Structure Activity Relationship (QSAR) of a fragment based model comprising:

identification means for identifying the fragments $[r_1\text{-}r_n]$ in the structure;

comparison means for comparing the or each fragment with one or more fragments in a model;

if the or each fragment substantially matches a fragment in the model, a first determination means for determining a first error measure between a contribution of the or each fragment and a contribution of the matching fragment in the model;

if the or each fragment does not substantially match a fragment in the model, a second determination means for determining which fragment in the model is the most similar to the or each fragment and determining a second error measure based on the similarity between the fragment and the most similar fragment; and

combining means for combining the output from the first and second determining means to generate a degree of separation between the structure and of the combined fragments in the model to estimate the distance from the structure to an application domain of said fragment based QSAR model.

Preferably the second measuring means comprises measuring means for measuring the degree of dissimilarity $(1\text{-}T_r)$ of the or each fragment from the most similar fragment in the model.

Also preferably the apparatus further comprises mapping means for determining a mapping X of the fragments in the structure such that: $X=[m_{kr}]$.

The above defined method and apparatus allow the calculation of a prediction even if there is the problem of missing fragments and allows a level of uncertainty (error) to be determined for the prediction. Other advantages are identified in the description.

Reference will now be made, by way of example, to the accompanying drawings in which:

Figure 1 is a diagram of an example of a chemical structure showing a number of fragments which is used to illustrate certain facets of the invention,

Figure 2 is a diagram showing the structures for two specific molecules (7-Methylthiopteridine and Fenitrothion),

Figure 3 is a table showing the contribution of fragments generated from the molecule of 7-Methylthiopteridine,

Figure 4 is a table showing the contribution of fragments generated from the molecule of Fenitrothion,

Figure 5 is a diagram showing the original and replacement fragments for 7-Methylthiopteridine,

Figure 6 is a diagram showing the original and replacement fragments for Fenitrothion.

QSARs are a mathematical relationship linking chemical structure and properties of that chemical in a quantitative manner for a series of compounds.

Methods such as regression and pattern recognition techniques are often adopted. Regression analysis is the use of statistical methods for modelling a set of dependant variable in terms of combinations of predictors. Methods such as multiple linear regression (MLR) and partial least squares (PLS) are included.

Pattern recognition is the identification of patterns in large data sets using mathematical analysis.

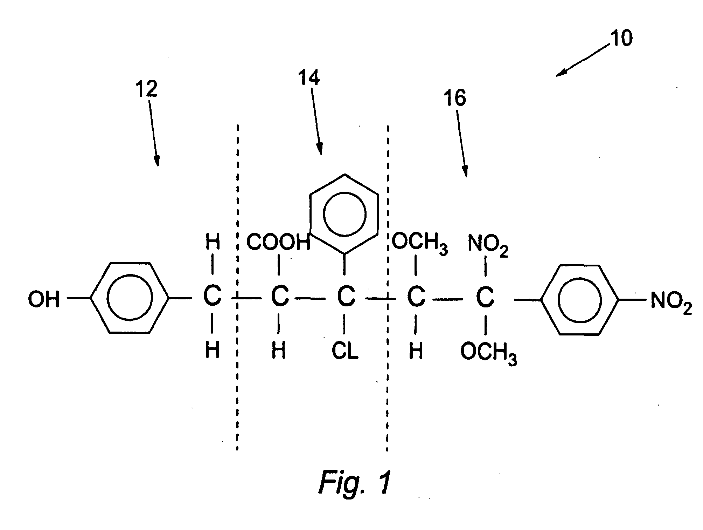

Referring initially to figure 1, a structure $k$ (10 in figure 1) is shown. The structure has an experimentally determined activity ($y$). Mathematically this is represented as:

$$y = \left[y_k\right] \quad \text{------}1$$

In addition the structure $k$ (10) has a number of fragments $r$ (12, 14, 16 in figure 1). The number of times a specific fragment $r$ occurs in a structure $k$ is given by the mapping count X of the fragments. Mathematically this may be expressed as:

$$X = \left[m_{kr}\right] \quad \text{--------}2$$

A multivariant linear regression process is used to model the activity.

$$Y \approx a_0 + \sum_r a_r m_r \quad ------3$$

Where $a_0$ is a constant and $a_r$ is the contribution for the variable $m_r$ and $m_r$ varies over the $m$ fragments. The values of $a_0$ and $a_r$ are determined that best fit the data.

The regression calculation also produces an "error weight" coefficient $e_r$ for each fragment $r$, from the diagonal of the inverse of the centred matrix. This can be expressed mathematically as:

$$e_r = E_{rr} --------\ 4$$

where:

$$E = \left( X^T X - \frac{1}{N} \left( \sum_{k=1}^{N} \mathbf{x}_k \right) \left( \sum_{k=1}^{N} \mathbf{x}_k^T \right) \right)^{-1} \quad -------\ 5$$

$N$ is the number of structures which have been trained on in the model; and is the row of X containing the mappings for structure $k$.

The QSAR model has a statistical variance $\sigma^2$ and the coefficient $e_r$ for each fragment is an indication of statistical variance in $a_r$ relative to that of the model. Thus it can be concluded that a mapping count of $m_r$ for fragment $r$ will contribute the following to the statistical variance of the predicted activity of the structure:

$$m_r^2 e_r \sigma^2 --------6$$

It should be noted that this is a somewhat simplified contribution which does not take into account correlation between different fragments' mapping. The contribution is intended to indicate the isolated effect of a single fragment. In the present invention this statistical variance is an important feature as it is representative of a "distance from the model" of a specific fragment. This is so as a high value of $e_r$ corresponds to only a little variation of the data set with respect to $m_r$.

When applying a fragment type QSAR model to a new structure there is a good chance that some (and maybe all) of the fragments are not present in the model. In this case a fragment in the structure is compared to a fragment in the model which is the most similar to it. The similarity is based on certain atom/bond configurations and may be expressed in terms of a coefficient, for example the Tanimoto coefficient T. The Tanimoto coefficient is a measure of similarity and has a value between 0 and 1 or 0% and 100%, depending on preference. Different measures of similarity can be used and the method of calculation is independent of the methods of similarity measurement.

The dissimilarity (1- $T_r$) of a fragment relative to the most similar fragment in the model, gives rise to another source of uncertainty. This uncertainty can also be interpreted as a "distance from the model" for a specific fragment.

This invention presents a means by which the two above mentioned uncertainties are combined for all fragments of a new structure to provide a single measure $d_{fm}$. This measure $d_{fm}$ is a measure of the distance of the structure from the domain of the fragment model. For a fragment-based model domain is the set of fragments. In a preferred method the measure can be determined from combining the "error weight" uncertainty and the dissimilarity uncertainty. This can be represented mathematically as:

$$d_{fm}^2 = \frac{1}{N} \sum_{r=1}^{N} (1 - T_r) m_r^2 e_r _____7$$

Where the sum is for all $N$ fragments occurring in the structure and $T_r$ is the similarity of fragment r in the structure to its closest match in the model.

The expression in equation 7 relates to a "squared distance measure" as it involves squared factors $m^2$ and $E$. This is consistent with how regression measures $r^2$ and $q^2$ are used to determine standard Euclidian distance in other areas of statistical analysis.

The method described above provides in non mathematical terms that only fragments that are <u>not</u> in the model contribute to $d_{fm}{}^2$; or mathematically if the similarity is equal to 1 (i.e. the fragment is the same) then this element of the equation will be zero (1-1=0), so will not contribute to the distance. If all fragments of a particular structure are in the model then the value of $d_{fm}{}^2$ will inevitably be zero.

The above method provides a method in which there is a clear definition of a domain of applicability for fragment-based QSAR models and the distance (or degree of error) from the domain is clearly identified. determination of the properties of a chemical structure which is analysed by this type of fragment based model. This in turn will mean that chemists wishing to design and generate new chemical structures for whatever purpose will have a more accurate means of prediction.

The method described herein suggests a set of mathematical representations all of which are by way of example only. Other ways of determining the error weight uncertainty for each fragment and of determining the dissimilarity uncertainty of each fragment and for combining them are equally valid, as will be appreciated by those skilled in the art.

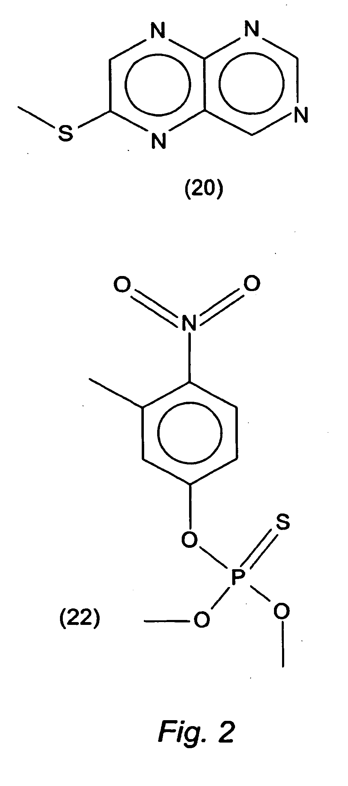

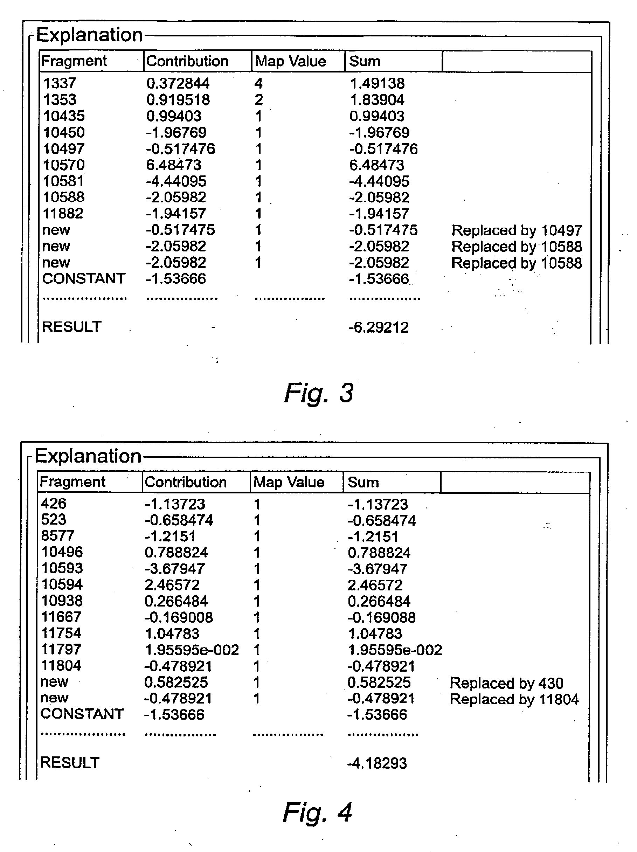

An example of use of the model is now described. During the external test set validation process using a fragment-based model for two molecules, not all fragments were found in the model. The structures for these molecules are represented in Figure 2. Calculated values for both compounds based on the summary of contribution for all fragments generated from the molecules are shown in the tables in Figure 3 and 4.

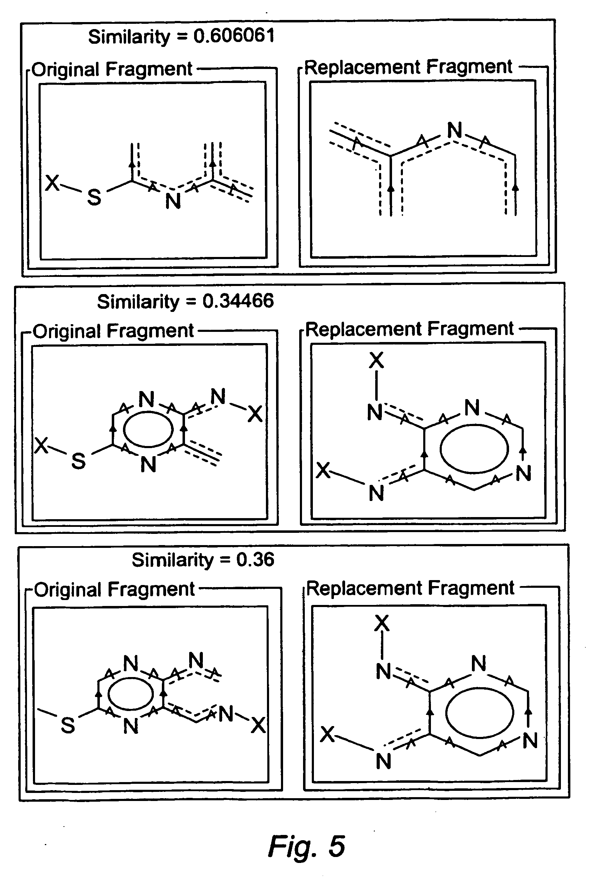

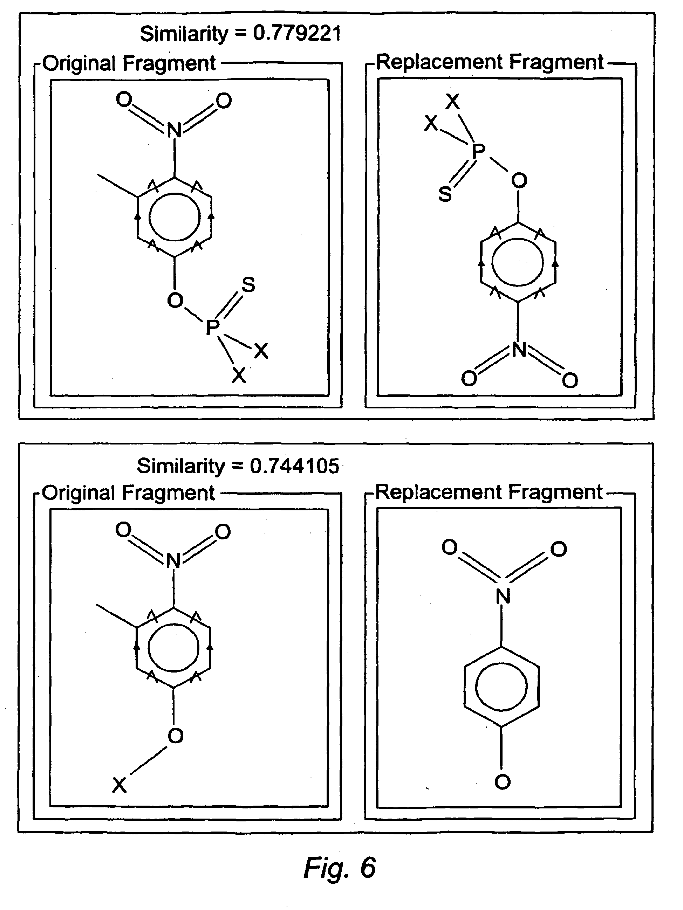

Both structures contain approximately the same number of fragments (12 and 13). For 7-Methylthiopteridine there are three fragments, which were not found in the model and have been replaced with the most similar fragment from the model. For Fenitrothion there are two such fragments. The original fragments and replacements are presented in Figure 5 and 6 respectively.

It is important to now determine what prediction can be trusted and which can not. To determine this it is necessary to calculate the distance from the domain using formula (7) based on the statistics presented below in Table 1.

**Table 1.**

| Compound | Num.of Fragments | Replacement Fragments ID | Similarity to the original fragment | Mapping value | Error Weight | Contribution | dfm$^2$ |
|---|---|---|---|---|---|---|---|
| 7-Methylthio-pteridine | 12 | 10497 | 0.606061 | 1 | 1.351 | 0.532211 | |
| | | 10588 | 0.34466 | 1 | 167.9 | 110.0315 | |
| | | 10588 | 0.36 | 1 | 167.9 | 107.456 | |
| | | | | | | 218.0197 | 18.16832 |
| Fenitrothion | 13 | 430 | 0.744186 | 1 | 0.768 | 0.196465 | |
| | | 11804 | 0.779221 | 1 | 2.122 | 0.468493 | |
| | | | | | | 0.664958 | 0.051151 |

The values of $d_{fm}{}^2$ are calculated below as follows:

$$d_{fm}{}^2 \text{ (7-Methylthiopteridine )} = (1/12) * [ (1-0.606061) * 1^2 * 1.351 + (1-0.34466) * 1^2 * 167.9 + (1-0.36) * 1^2 * 167.9 ] =$$

$$= (1/12) * [0.5322 + 110.0 + 107.5] = 218.0 / 12 = 18.17$$

$$d_{fm}^2 \text{ (Fenitrothion )} = (1/13) * [ (1-0.744186) * 1^2 *$$
$$0.7680 + (1-0.779221) * 1^2 *2.122 ]$$
$$= (1/13) * [0.1965 + 0.4685] = 0.6650 / 13 = 0.05115$$

Comparison between these two values (18.17 and 0.05115) provides the answer: Fenitrothion is very close to the domain of the model whilst 7-Methylthiopteridine is very far from this domain. The prediction value for the 7-Methylthiopteridine cannot therefore be taken in account. This result corresponds to the experimental values/residuals for these structures:

7-Methylthiopteridine: Experimental value is - 1.55, predicted value is -6.292, and the absolute residual value is 4.742 (305.9%). Fenitrothion: Experimental value is -4.04, predicted value is - 4.183, and the absolute residual value is 0.1429 (3.54%).

The new simple and quantitative method for assessment of domain of applicability for the fragment-based QSAR models is thus proposed. The model takes into account the total number of fragments generated from the structure, availability the most similar fragments in the model and their contributions values. Implementation of this method allows the definition of statistical significance of the predictions and solving the problem of missing fragments, which is crucial for fragment-based QSAR models.

**Claims**

1.  A computer implemented method for estimation of the distance from an application domain of a fragment based model by means of a fragment - based Quantitative Structure Activity Relationship, QSAR, model, the steps comprising;

    identifying the fragments $r_1$ to $r_n$ in a structure;
    comparing the or each fragment with one or more fragments in the model;
    if the or each fragment substantially matches a fragment in the model determining a first error measure between a contribution of the or each fragment and a contribution of the matching fragment in the model;
    if the or each fragment does not substantially match a fragment in the model determining which fragment in the model is the most similar to the or each fragment and determining a second error measure based on the similarity between the fragment and the most similar fragment; and
    combining the first error measure and the second error measure to generate a degree of separation between the structure and of the combined fragments in the model to estimate the distance from the structure to an application domain of said fragment based QSAR model.

2.  The method of claim 1, wherein the step of identifying the fragments *r* further comprises for the or each structure k determining matrix X of mapping count $m_r$ for fragments r for the structure k such that

$$X = \left[ m_{kr} \right].$$

3.  The method of claim 1 or claim 2, wherein the step of determining the first error measure comprises calculating an error weight coefficient e, in accordance with

$$e_r = E_{rr}$$

    where

$$E = \left( X^T X - \frac{1}{N} \left( \sum_{k=1}^{N} \mathbf{x}_k \right) \left( \sum_{k=1}^{N} \mathbf{x}_k^T \right) \right)^{-1}$$

$N$ is the number of structures which have been trained on in the model;
$x_k = [m_{kr}]$ is the row of X containing the mappings for structure k; and
T is the standard notation for the transpose of a matix.

4. The method of any preceding claim, wherein the step of determining the second error measure comprises measuring the degree of dissimilarity, $1-T_r$, of the or each fragment from the most similar fragment in the model where $T_r$ is a similarity coefficient.

5. The method of claim 4, wherein the step of combining comprises combining the error weight coefficient and the degree of dissimilarity.

6. The method of any preceding claim wherein the distance from the domain is estimated from the step of combining the first and second error measurements.

7. The method of any preceding claim wherein the distance of the structure from the domain of the QSAR fragment model $d_{fm}$ is estimated in accordance with:

$$d_{fm}^2 = \frac{1}{N} \sum_{r=1}^{N} (1 - T_r) m_r^2 e_r$$

where $T_r$ is the measure of similarly and $m_r$ is the mapping count for fragment r.

8. A method of determining the match between a structure and a model of a structure including the method of estimating distance from a domain in accordance with any one of claims 1 to 7.

9. A computer apparatus for estimating the distance from an application domain Quantitative Structure Activity Relationship of a fragment based model comprising:

identification means for identifying the fragments $r_1$ to $r_n$ in the structure;
comparison means for comparing the or each fragment with one or more fragments in a model;
if the or each fragment substantially matches a fragment in the model, a first determination means for determining a first error measure between a contribution of the or each fragment and a contribution of the matching fragment in the model;
if the or each fragment does not substantially match a fragment in the model, a second determination means for determining which fragment in the model is the most similar to the or each fragment and determining a second error measure based on the similarity between the fragment and the most similar fragment; and
combining means for combining the output from the first and second determining means to generate a degree of separation between the structure and of the combined fragments in the model to estimate the distance from the structure to an application domain of said fragment based QSAR model.

10. The apparatus of claim 9, wherein the second measuring means comprises measuring means for measuring the degree of dissimilarity $1-T_r$ of the or each fragment from the most similar fragment in the model.

11. The apparatus of claim 9 or claim 10 further comprises mapping means for determining matrix X of the mapping count of the fragments r in the structure k such that:

$$X = [m_{kr}]$$

**Patentansprüche**

1. Ein computerimplementiertes Verfahren zur Einschätzung der Distanz von einem Anwendungsbereich eines fragmentbasierten Modells mittels eines fragmentbasierten Modells quantitativer Struktur-Wirkungsbeziehungen (QSAR-Modell), wobei die Schritte Folgendes beinhalten:

   Identifizieren der Fragmente $r_1$ bis $r_n$ in einer Struktur;
   Vergleichen des oder jedes Fragments mit einem oder mehreren Fragmenten in dem Modell;
   wenn das oder jedes Fragment im Wesentlichen mit einem Fragment in dem Modell übereinstimmt, Bestimmen eines ersten Fehlermaßes zwischen einem Beitrag des oder jedes Fragments und einem Beitrag des übereinstimmenden Fragments in dem Modell;
   wenn das oder jedes Fragment nicht wesentlich mit einem Fragment in dem Modell übereinstimmt, Bestimmen, welches Fragment in dem Modell dem oder jedem Fragment am ähnlichsten ist, und Bestimmen eines zweiten Fehlermaßes auf der Basis der Ähnlichkeit zwischen dem Fragment und dem ähnlichsten Fragment; und
   Kombinieren des ersten Fehlermaßes und des zweiten Fehlermaßes, um einen Grad an Trennung zwischen der Struktur und den kombinierten Fragmenten in dem Modell zu erzeugen, um die Distanz von der Struktur zu einem Anwendungsbereich des fragmentbasierten QSAR-Modells einzuschätzen.

2. Verfahren gemäß Anspruch 1, wobei der Schritt des Identifizierens der Fragmente $r$ des Weiteren für die oder jede Struktur k das Bestimmen einer Matrix X der Abbildungszahl $m_r$ für Fragmente r für die Struktur k beinhaltet, so dass

$$X = [m_{kr}].$$

3. Verfahren gemäß Anspruch 1 oder Anspruch 2, wobei der Schritt des Bestimmens des ersten Fehlermaßes das Berechnen eines Fehlergewichtskoeffizienten $e_r$ gemäß

$$e_r = E_{rr}$$

beinhaltet, wobei

$$E = \left( X^T X - \frac{1}{N} \left( \sum_{k=1}^{N} x_k \right) \left( \sum_{k=1}^{N} x_k^T \right) \right)^{-1}$$

   $N$ die Anzahl von Strukturen ist, die beim Anlernen in dem Modell verwendet wurden;
   $x_k = [m_{kr}]$ die Zeile von X ist, die die Abbildungen für die Struktur $k$ enthält; und
   T die Standardschreibweise für die Transponierte einer Matrix ist.

4. Verfahren gemäß einem der vorhergehenden Ansprüche, wobei der Schritt des Bestimmens des zweiten Fehlermaßes das Messen des Grads an Verschiedenheit $1 - T_r$ des oder jedes Fragments von dem ähnlichsten Fragment in dem Modell beinhaltet, wobei $T_r$ ein Ähnlichkeitskoeffizient ist.

5. Verfahren gemäß Anspruch 4, wobei der Schritt des Kombinierens das Kombinieren des Fehlergewichtskoeffizienten und des Grads an Verschiedenheit beinhaltet.

6. Verfahren gemäß einem der vorhergehenden Ansprüche, wobei die Distanz von dem Definitionsbereich aus dem Schritt des Kombinierens der ersten und der zweiten Fehlermessung eingeschätzt wird.

7. Verfahren gemäß einem der vorhergehenden Ansprüche, wobei die Distanz der Struktur von dem Definitionsbereich des QSAR-Fragmentmodells $d_{fm}$ gemäß dem Folgenden eingeschätzt wird:

$$d_{fm}^2 = \frac{1}{N} \sum_{r=1}^{N} (1 - T_r) m_r^2 e_r$$

wobei $T_r$ das Maß der Ähnlichkeit und $m_r$ die Abbildungszahl für das Fragment r ist.

8. Ein Verfahren zum Bestimmen der Übereinstimmung zwischen einer Struktur und einem Modell einer Struktur, das das Verfahren des Einschätzens der Distanz von einem Definitionsbereich gemäß einem beliebigen der Ansprüche 1 bis 7 umfasst.

9. Eine Computerausrüstung zum Einschätzen der Distanz von einer quantitativen Struktur-Wirkungsbeziehung des Anwendungsbereichs eines fragmentbasierten Modells, die Folgendes beinhaltet:

ein Identifizierungsmittel zum Identifizieren der Fragmente $r_1$ bis $r_n$ in der Struktur;
ein Vergleichsmittel zum Vergleichen des oder jedes Fragments mit einem oder mehreren Fragmenten in einem Modell;
wenn das oder jedes Fragment im Wesentlichen mit einem Fragment in dem Modell übereinstimmt, ein erstes Bestimmungsmittel zum Bestimmen eines ersten Fehlermaßes zwischen einem Beitrag des oder jedes Fragments und einem Beitrag des übereinstimmenden Fragments in dem Modell;
wenn das oder jedes Fragment nicht wesentlich mit einem Fragment in dem Modell übereinstimmt, ein zweites Bestimmungsmittel zum Bestimmen, welches Fragment in dem Modell dem oder jedem Fragment am ähnlichsten ist, und Bestimmen eines zweiten Fehlermaßes auf der Basis der Ähnlichkeit zwischen dem Fragment und dem ähnlichsten Fragment; und
ein Kombinationsmittel zum Kombinieren des Ausgangs von dem ersten und dem zweiten Bestimmmittel, um einen Grad an Trennung zwischen der Struktur und den kombinierten Fragmenten in dem Modell zu erzeugen, um die Distanz von der Struktur zu einem Anwendungsbereich des fragmentbasierten QSAR-Modells einzuschätzen.

10. Ausrüstung gemäß Anspruch 9, wobei das zweite Messmittel ein Messmittel zum Messen des Grads an Verschiedenheit 1 - $T_r$ des oder jedes Fragments von dem ähnlichsten Fragment in dem Modell beinhaltet.

11. Ausrüstung gemäß Anspruch 9 oder Anspruch 10, die des Weiteren ein Abbildungsmittel zum Bestimmen der Matrix X der Abbildungszahl der Fragmente r in der Struktur k beinhaltet, so dass:

**X = [m$_{kr}$].**

**Revendications**

1. Une méthode implémentée par ordinateur destinée à l'évaluation de la distance depuis un domaine d'application d'un modèle basé sur un fragment au moyen d'un modèle de relation quantitative structure-activité, QSAR, basé sur un fragment, les étapes comprenant :

identifier les fragments $r_1$ à $r_n$ dans une structure ;
comparer le ou chaque fragment avec un ou plusieurs fragments dans le modèle ;
si le ou chaque fragment correspond substantiellement à un fragment dans le modèle, déterminer une première mesure d'erreur entre une contribution du ou de chaque fragment et une contribution du fragment correspondant dans le modèle ;
si le ou chaque fragment ne correspond pas substantiellement à un fragment dans le modèle, déterminer quel fragment dans le modèle est le plus similaire au ou à chaque fragment et déterminer une deuxième mesure d'erreur sur la base de la similarité entre le fragment et le fragment le plus similaire ; et
combiner la première mesure d'erreur et la deuxième mesure d'erreur pour générer un degré de séparation entre la structure et les fragments combinés dans le modèle pour évaluer la distance de la structure à un domaine d'application dudit modèle QSAR basé sur un fragment.

2. La méthode de la revendication 1, dans laquelle l'étape consistant à identifier les fragments *r* comprend en outre pour la ou chaque structure k déterminer une matrice X de compte de mappage $m_r$ pour des fragments r pour la structure k de telle sorte que

$$X = [m_{kr}].$$

3. La méthode de la revendication 1 ou de la revendication 2, dans laquelle l'étape consistant à déterminer la première mesure d'erreur comprend calculer un coefficient de poids d'erreur $e_r$ conformément à

$$e_r = E_{rr}$$

où

$$E = \left( X^T X - \frac{1}{N} \left( \sum_{k=1}^{N} x_k \right) \left( \sum_{k=1}^{N} x_k^T \right) \right)^{-1}$$

$N$ est le nombre de structures sur lesquelles un entraînement a été réalisé dans le modèle ;
$x_k = [m_{kr}]$ est la rangée de X contenant les mappages pour la structure $k$ ; et
T est la notation standard pour la transposée d'une matrice.

4. La méthode de n'importe quelle revendication précédente, dans laquelle l'étape consistant à déterminer la deuxième mesure d'erreur comprend mesurer le degré de dissimilarité, 1-$T_r$, du ou de chaque fragment d'après le fragment le plus similaire dans le modèle où $T_r$ est un coefficient de similarité.

5. La méthode de la revendication 4, dans laquelle l'étape consistant à combiner comprend combiner le coefficient de poids d'erreur et le degré de dissimilarité.

6. La méthode de n'importe quelle revendication précédente dans laquelle la distance depuis le domaine est évaluée d'après l'étape consistant à combiner les première et deuxième mesures d'erreur.

7. La méthode de n'importe quelle revendication précédente dans laquelle la distance de la structure depuis le domaine du modèle à fragment QSAR $d_{fm}$ est évaluée conformément à :

$$d_{fm}^2 = \frac{1}{N} \sum_{r=1}^{N} (1 - T_r) m_r^2 e_r$$

où $T_r$ est la mesure de similarité et $m_r$ est le compte de mappage pour un fragment r.

8. Une méthode pour déterminer la correspondance entre une structure et un modèle d'une structure incluant la méthode pour évaluer une distance depuis un domaine conformément à n'importe laquelle des revendications 1 à 7.

9. Un appareil informatique destiné à évaluer la distance depuis une relation quantitative structure-activité de domaine d'application d'un modèle basé sur un fragment comprenant :

un moyen d'identification destiné à identifier les fragments $r_1$ à $r_n$ dans la structure ;
un moyen de comparaison destiné à comparer le ou chaque fragment avec un ou plusieurs fragments dans un modèle ;

si le ou chaque fragment correspond substantiellement à un fragment dans le modèle, un premier moyen de détermination destiné à déterminer une première mesure d'erreur entre une contribution du ou de chaque fragment et une contribution du fragment correspondant dans le modèle ;

si le ou chaque fragment ne correspond pas substantiellement à un fragment dans le modèle, un deuxième moyen de détermination destiné à déterminer quel fragment dans le modèle est le plus similaire au ou à chaque fragment et déterminer une deuxième mesure d'erreur sur la base de la similarité entre le fragment et le fragment le plus similaire ; et

un moyen de combinaison destiné à combiner la sortie provenant du premier et du deuxième moyen de détermination pour générer un degré de séparation entre la structure et les fragments combinés dans le modèle pour évaluer la distance de la structure à un domaine d'application dudit modèle QSAR basé sur un fragment.

**10.** L'appareil de la revendication 9, dans lequel le deuxième moyen de mesure comprend un moyen de mesure destiné à mesurer le degré de dissimilarité $1-T_r$ du ou de chaque fragment d'après le fragment le plus similaire dans le modèle.

**11.** L'appareil de la revendication 9 ou de la revendication 10 comprend en outre un moyen de mappage destiné à déterminer une matrice X du compte de mappage des fragments r dans la structure k de telle sorte que :

$$X = [m_{kr}]$$

*Fig. 1*

(20)

(22)

*Fig. 2*

## Explanation

| Fragment | Contribution | Map Value | Sum | |
|---|---|---|---|---|
| 1337 | 0.372844 | 4 | 1.49138 | |
| 1353 | 0.919518 | 2 | 1.83904 | |
| 10435 | 0.99403 | 1 | 0.99403 | |
| 10450 | -1.96769 | 1 | -1.96769 | |
| 10497 | -0.517476 | 1 | -0.517476 | |
| 10570 | 6.48473 | 1 | 6.48473 | |
| 10581 | -4.44095 | 1 | -4.44095 | |
| 10588 | -2.05982 | 1 | -2.05982 | |
| 11882 | -1.94157 | 1 | -1.94157 | |
| new | -0.517475 | 1 | -0.517475 | Replaced by 10497 |
| new | -2.05982 | 1 | -2.05982 | Replaced by 10588 |
| new | -2.05982 | 1 | -2.05982 | Replaced by 10588 |
| CONSTANT | -1.53666 | | -1.53666 | |
| ................... | ................... | ................... | ................... | |
| RESULT | - | | -6.29212 | |

*Fig. 3*

## Explanation

| Fragment | Contribution | Map Value | Sum | |
|---|---|---|---|---|
| 426 | -1.13723 | 1 | -1.13723 | |
| 523 | -0.658474 | 1 | -0.658474 | |
| 8577 | -1.2151 | 1 | -1.2151 | |
| 10496 | 0.788824 | 1 | 0.788824 | |
| 10593 | -3.67947 | 1 | -3.67947 | |
| 10594 | 2.46572 | 1 | 2.46572 | |
| 10938 | 0.266484 | 1 | 0.266484 | |
| 11667 | -0.169008 | 1 | -0.169088 | |
| 11754 | 1.04783 | 1 | 1.04783 | |
| 11797 | 1.95595e-002 | 1 | 1.95595e-002 | |
| 11804 | -0.478921 | 1 | -0.478921 | |
| new | 0.582525 | 1 | 0.582525 | Replaced by 430 |
| new | -0.478921 | 1 | -0.478921 | Replaced by 11804 |
| CONSTANT | -1.53666 | | -1.53666 | |
| ................... | ................... | ................... | ................... | |
| RESULT | | | -4.18293 | |

*Fig. 4*

Fig. 5

Fig. 6

# EP 1 708 108 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **R.S Sheridan ; BP Fenston ; VN Maiorov ; SK Kearsley.** Similarity of molecules in the training set is a good discriminator for prediction accuracy. *QSAR. J. Chem. Inf. Comput.SCI.,* 2004, 1912-1928

19